# EUROPEAN PATENT APPLICATION

(11) **EP 4 159 838 A1**
(43) Date of publication of application: **05.04.2023**
(21) Application number: 21813340.3
(22) Date of filing: 27.05.2021
(51) Int. Cl.: C12M 3/00, C12N 5/071

(54) **MASS PRODUCTION METHOD OF UNIFORM SIZE CELL AGGREGATE**

(30) Priority: 28.05.2020 JP 2020093447
(71) Applicant: Orizuru Therapeutics, Inc., Fujisawa-shi Kanagawa 251-8555 (JP); Toyo Seikan Group Holdings, Ltd., Shinagawa-ku Tokyo 141-8627 (JP)
(72) Inventor: YAMAURA Junji, Fujisawa-shi, Kanagawa 251-0012 (JP); ITO Ryo, Fujisawa-shi, Kanagawa 251-0012 (JP); TOYODA Taro, Kyoto-shi, Kyoto 606-8501 (JP); KONAGAYA Shuhei, Kyoto-shi, Kyoto 606-8501 (JP); SUENAGA Ryo, Yokoham-shi, Kanagawa 240-0062 (JP)
(74) Representative: Jones, Nicholas Andrew
(86) International application number: PCT/JP2021/020135
(87) International publication number: WO 2021/241668

(57) **Abstract**

An object of the present invention is to provide a novel approach capable of conveniently producing a large quantity of substantially uniform size cell aggregates. Provided is a method for producing cell aggregates using a cell culture bag, the cell culture bag having a lower face comprising a plurality of recesses, the method comprising the steps of: (1) adding cells and a medium to the cell culture bag, stirring the contents, and culturing the cells while applying a pressure to the cell culture bag; and (2) recovering the formed cell aggregates after the completion of culture.

## Description

### Technical Field

The present invention relates to a method for conveniently producing a large quantity of substantially uniform size cell aggregates.

### [Background of Invention]

In recent years, the mass culture of cells of interest in an artificial environment has been practiced in the fields of gene therapy, regenerative therapy, and the like. An exemplary approach involves using a plate with one or more recesses formed therein (generally called "well plate"), introducing cells and a culture medium into each individual recess, and culturing the cells. However, this approach has the risk of contamination with foreign matter because the recesses are exposed to the atmosphere. Furthermore, cells and a culture medium must be injected and recovered on a recess basis, and this complicates procedures. Thus, mass culture at a large scale places large burdens on workers.

By contrast, an approach of performing mass culture in a closed manner using a gas permeable sac-like container (also referred to as a "bag") with a plurality of recesses formed on a lower face has been developed and reported (e.g., Patent Literature 3 mentioned later). This approach improves sealability as compared with the case of using the well plate mentioned above, and has the advantage that the risk of contamination with foreign matter can be reduced.

On the other hand, closed culture using such a bag may vary in the size, number, or the like of formed cultures (more specifically, cell aggregates) among recesses, because the number, position, or the like of cells placed in each recess cannot be adjusted. This requires complicated procedures of, for example, detaching cells in each recess and arranging the cells at appropriate positions, while limiting movement among recesses during culture. Thus, mass culture at a large scale places large burdens on workers.

Hence, a closed culture method capable of conveniently producing a large quantity of substantially uniform cell cultures has been desired in the art.

Heretofore, the following approaches have been developed and reported as closed culture methods using a gas permeable sac-like container.

Patent Literature 1 discloses a method for culturing adhesive cells, comprising adding the adhesive cells and a culture medium to a sac-like container having a container wall serving as a cell culture face, and elevating the internal pressure of the sac-like container by pressing so that the contents are prevented from moving to promote the adhesion of the cells to the cell culture face or to suppress the detachment of the cells that have adhered thereto.

Patent Literature 2 discloses a cell culture method comprising placing a flexible culture container supplemented with cells and a culture medium on a placement face, continuing culture in a state where an outer face of the flexible culture container contacted with the placement face is deformed and a plurality of recesses are formed by the application of a pressure to the flexible culture container, and releasing the pressure against the flexible culture container after formation of cell spheroids in the recesses to flatten the recesses formed on the outer face of the container, followed by further culture. Patent Literature 2 states that the recesses are flattened by releasing the pressure applied during culture, because the maintenance of the recesses during culture might be inconvenient to the circulation of a medium or the diffusion of a gas, and says that such flattened recesses can expand a region (area) for use in cell culture.

Patent Literature 3 discloses a method for culturing cell spheroids using a cell culture bag with a plurality of recesses formed on a lower face, and states that this method requires a procedure of detaching the spheroids that have adhered to inner faces of the recesses for forming one spheroid in each recess and for improving the proliferation and/or differentiation induction efficiency of cells, and in the detachment step, the plurality of recesses are blocked in order to prevent the movement of the spheroids among the recesses.

### Citation List

### Patent Literature

Patent Literature 1: Japanese Patent Laid-Open No. 2016-86774
Patent Literature 2: WO2016/208526
Patent Literature 3: Japanese Patent Laid-Open No. 2019-118319

### Summary of Invention

### Technical Problem

All the conventional techniques described above neither disclose nor suggest convenient production of a large quantity of substantially uniform size cell aggregates.

Accordingly, an object of the present invention is to provide a novel approach capable of conveniently producing a large quantity of substantially uniform size cell aggregates.

### Solution to Problem

The present inventors have conducted diligent studies to attain the object and consequently found that substantially uniform size cell aggregates are formed in individual recesses by adding cells and a culture medium to a gas permeable flexible cell culture bag comprising a plurality of recesses on a lower face, and culturing the cells while applying a pressure to the cell culture bag. The present inventors have also found that a large quantity of the formed cell aggregates can be taken out of the individual recesses by a convenient operation after the completion of culture and can be conveniently recovered from the cell culture bag. The present invention is based on these novel findings and encompasses the following embodiments.
[1] A method for producing cell aggregates using a cell culture bag,
   the cell culture bag having an upper face and a lower face and comprising a plurality of recesses on the lower face,
   the method comprising the steps of:
      (1) adding cells and a medium to the cell culture bag, stirring the contents, and culturing the cells while applying a pressure in at least one direction to the cell culture bag; and
      (2) recovering the formed cell aggregates after the completion of culture.
[2] The method according to [1], wherein 100000 to 1000000 cell aggregates are recovered.
[3] The method according to [1] or [2], wherein the recovered cell aggregates have a substantially uniform particle size.
[4] The method according to any of [1] to [3], wherein 70% or more of the recovered cell aggregates have a particle size within ±10% of the median particle size of the cell aggregates.
[5] The method according to any of [1] to [4], wherein 70% or more of the recovered cell aggregates have a particle size of 140 µm to 160 µm.
[6] The method according to any of [1] to [5], wherein 200 to 2000 cells are included per recovered cell aggregate.
[7] The method according to any of [1] to [6], wherein the cells are frozen-thawed cells.
[8] The method according to any of [1] to [7], wherein the cells are insulin-secreting cells.
[8a] The method according to [8], wherein the insulin-secreting cells are insulin-producing cells.
[9] The method according to any of [1] to [8], wherein the cell aggregates are recovered by flipping the cell culture bag vertically.
[10] The method according to any of [1] to [9], wherein the cell aggregates are recovered by injecting air to the cell culture bag.
[11] The method according to any of [1] to [10], wherein the step of culturing the cells while applying a pressure in at least one direction to the cell culture bag is the step of culturing the cells while applying a pressure to the cell culture bag from above, from below or from both above and below.
[12] The method according to [11], wherein the step of culturing the cells while applying a pressure in at least one direction to the cell culture bag is the step of culturing the cells while applying a pressure to the cell culture bag from above.
[13] The method according to any of [1] to [12], wherein a depth:diameter ratio of the recesses is 1:1.5 to 2.5.
[14] The method according to any of [1] to [13], wherein the recesses have a spherical cap shape.
[15] The method according to any of [1] to [14], wherein a pitch of the recesses is 0.35 mm to 0.49 mm.
[16] The method according to any of [1] to [15], wherein a depth of the recesses is 150 µm to 220 µm.
[17] The method according to any of [1] to [16], wherein a liquid depth of the cell culture bag is 1.5 mm to 6 mm.
[18] The method according to any of [1] to [17], wherein the cells are added in an amount of 1 × 10⁵ to 1 × 10⁷ cells/mL to the cell culture bag.
[19] The method according to any of [1] to [18], wherein the cells are added in an amount of 1 × 10⁴ to 5 × 10⁶ cells/cm² to the cell culture bag.
[20] The method according to any of [1] to [19], wherein a medium is further added after cell aggregate formation.
[21] The method according to any of [1] to [20], wherein the lower face comprises 100000 to 1000000 recesses.
[22] The method according to any of [1] to [21], wherein the recesses are included at 1 to 1000 recesses/cm² per lower face unit area.
[23] A cell aggregate population for transplantation, wherein 70% or more of the cell aggregates have a particle size of 140 µm to 160 µm.
[24] The cell aggregate population according to [23], comprising 100000 to 1000000 cell aggregates.
[25] The cell aggregate population according to [23] or [24], comprising insulin-secreting cells.
[25a] The cell aggregate population according to [25], wherein the insulin-secreting cells are insulin-producing cells.
[26] A cell culture bag comprising 100000 to 1000000 cell aggregates having a substantially uniform particle size.
[27] The cell culture bag according to [26], wherein 70% or more of the cell aggregates have a particle size of 140 µm to 160 µm.
[28] The cell culture bag according to [26] or [27], wherein the cell aggregates comprise insulin-secreting cells.
[28a] The cell culture bag according to [28], wherein the insulin-secreting cells are insulin-producing cells.

The present specification encompasses the contents described in the specification and/or drawings of Japanese Patent Application No. 2020-93447 filed on May 28, 2020 on which the priority of the present application is based.

All publications, patents and patent applications cited herein are incorporated herein by reference in their entirety.

### Advantageous Effects of Invention

The present invention can provide a novel approach capable of conveniently producing a large quantity of substantially uniform size cell aggregates.

### Brief Description of Drawings

[Figure 1] Figure 1 is a schematic view showing one example of a cell culture bag that can be used in the present invention. Figure 1(a) is a perspective view, and Figure 1(b) is a transparent perspective view.
[Figure 2] Figure 2 is a schematic view showing another example of a cell culture bag that can be used in the present invention. Figure 2(a) is a perspective view, and Figure 2(b) is a transparent perspective view.
[Figure 3] Figure 3(1) is a perspective view showing one example of a placement table for placing a cell culture bag that can be used in the present invention. Figure 3(2) is a schematic view showing that recesses are formed on a lower face of the cell culture bag by placing the cell culture bag on the placement table, and applying a pressure to the cell culture bag sandwiched between the placement table and a pressing member.
[Figure 4] Figure 4 is a cross-sectional schematic view of a culture apparatus that can be used in the present invention. Figure 4(a) shows an example having a placement face with formed openings to accept recesses of a cell culture bag, and Figure 4(b) shows an example having a flat placement face.
[Figure 5] Figure 5 is a perspective view showing another example of a culture apparatus that can be used in the present invention.
[Figure 6] Figure 6 is a photographic view of cell aggregates produced by the method of the present invention.
[Figure 7] Figure 7 is a graph showing a diameter distribution of recovered cell aggregates produced from insulin-producing cells.
[Figure 8] Figure 8 is a graph showing a diameter distribution of recovered cell aggregates produced from human iPS cells. Figure 8(A) shows the cell aggregates produced without applying a pressure during culture (control), and Figure 8(B) shows the cell aggregates produced by applying a pressure during culture.

### Description of Embodiments

### 1. Cell culture bag

As used herein, the "cell culture bag" means a culture container formed in a sac-like shape using a gas permeable flexible film material. The cell culture bag has a shape having at least a lower face comprising recesses, and preferably further has a shape having an upper face opposed to the lower face.

The "gas permeability" means a property of allowing a gas to pass through a flexible film material. In the cell culture bag according to the present invention, the transmission rate of oxygen measured at a test temperature of 37°C in Determination of Gas Transmission Rate of JIS K 7126 is preferably 5000 mL/(m²·day/atm) or more.

Examples of the flexible film material that can be used in the cell culture bag include, but are not limited to, films of resins such as polyethylene, polypropylene, ethylene-vinyl acetate copolymers, polyester, silicone elastomers, polystyrene elastomers, and tetrafluoroethylene-hexafluoropropylene copolymers (FEP). These films may each be used in the form of a single-layer film or may be used in the form of a multilayer (e.g., two-layer or three-layer) film in which materials of the same type or different types are laminated. In the case of forming the cell culture bag by stacking and heat-sealing two films, it is preferred to have a layer that functions as a sealant layer in consideration of heat sealability. For example, the flexible film material can have a configuration of three layers, an inner layer, a base layer, and an outer layer, in order from the inner side of the cell culture bag. The base layer and the inner layer are preferably each constituted using a material having high gas permeability, heat sealability, and transparency. The inner layer is preferably constituted by a material having low cytotoxicity in addition to the characteristics described above. For example, a polyethylene resin such as linear low density polyethylene (LLDPE), very low density polyethylene (VLDPE) or ultra low density polyethylene (ULDPE), low density polyethylene (LDPE), or a blend thereof can be suitably used as such a material. A polyethylene resin having a density of 0.886 g/cm³ to 0.93 g/cm³ is suitable for the outer layer. The outer layer may be appropriately omitted. A surface serving as an inner face of the cell culture bag (at least the surface of the lower face) is preferably subjected to low cell adhesion treatment in order to facilitate gathering cells to the center of the bottom of the recesses. Examples of the low cell adhesion treatment include coating with phospholipid polymers, polyvinyl alcohol derivatives, surfactants, or albumin. The thickness of the film can be on the order of 50 to 300 µm, preferably 80 to 160 µm and may be the same thickness or different thicknesses between the upper face and the lower face of the cell culture bag.

The number of recesses carried by the lower face of the cell culture bag may be determined depending on the size of the cell culture bag or the desired number of cell aggregates, and can be appropriately selected from the range of, for example, 10 to 1000000, 100 to 1000000, 1000 to 1000000, or 10000 to 1000000, preferably the range of 100000 to 1000000 or 300000 to 1000000.

The shape of the recesses is not particularly limited and is preferably a shape that facilitates gathering cells to the bottom of the recesses. Examples of the shape of the recesses include a spherical cap shape and a shape having a mortar-like (conical) depression. The depth:diameter ratio of the recesses is preferably 1:1 to 4, more preferably 1:1.5 to 2.5, further preferably 1:1.8 to 2.2, in order to facilitate gathering cells to the bottom of the recesses. For example, in one embodiment, the depth of the recesses can be 100 µm to 1000 µm, for example, 150 µm to 220 µm, and the opening size (diameter) thereof can be 300 µm to 1500 µm, for example, 225 µm to 550 µm.

The recesses are preferably arranged in a staggered pattern so as to maximize an area occupied by the recesses in the lower face, and may be arranged, if necessary, in a reticular pattern. The center-to-center spacing (pitch) between adjacent recesses 4 can be 0.35 mm to 0.49 mm, preferably 0.38 mm to 0.45 mm. Alternatively, the spacing between the outlines of adjacent recesses can be set to larger than 0 to 1 mm, preferably 0.05 to 0.1 mm. The recesses can be established at 1 to 1000 recesses/cm², for example, 30 to 800 recesses/cm² or 60 to 700 recesses/cm² per lower face unit area.

The size of the cell culture bag is not particularly limited and can be, for example, 10 to 1000 mm long, for example, 50 to 500 mm long, and 10 to 1000 mm wide, for example, 50 to 500 mm wide. The thickness of the cell culture bag is not particularly limited and can be a thickness that enables a liquid depth (distance from the inner surface of a flat face (site except for the recesses) of the lower face film that comes in contact with a culture solution to the inner surface of the upper face film) to be on the order of 1 mm to 20 mm, preferably 2 mm to 8 mm, in applying a pressure to the cell culture bag supplemented with a culture medium.

The cell culture bag can have a tubular member allowing a medium, cells, and the like to flow (hereinafter, this member is also referred to as a "port"). The port can be formed from, for example, a thermoplastic resin such as polyethylene, polypropylene, vinyl chloride, a polyethylene elastomer, or a tetrafluoroethylene-hexafluoropropylene copolymer (FEP).

For the form of the "cell culture bag" according to the present invention, the forms of sac-like culture containers formed using conventional gas permeable flexible film materials known in the art (Japanese Patent No. 5344094, WO2016/208526, Japanese Patent Laid-Open No. 2016-86774, Japanese Patent Laid-Open No. 2019-118319, etc.) can be referred to.

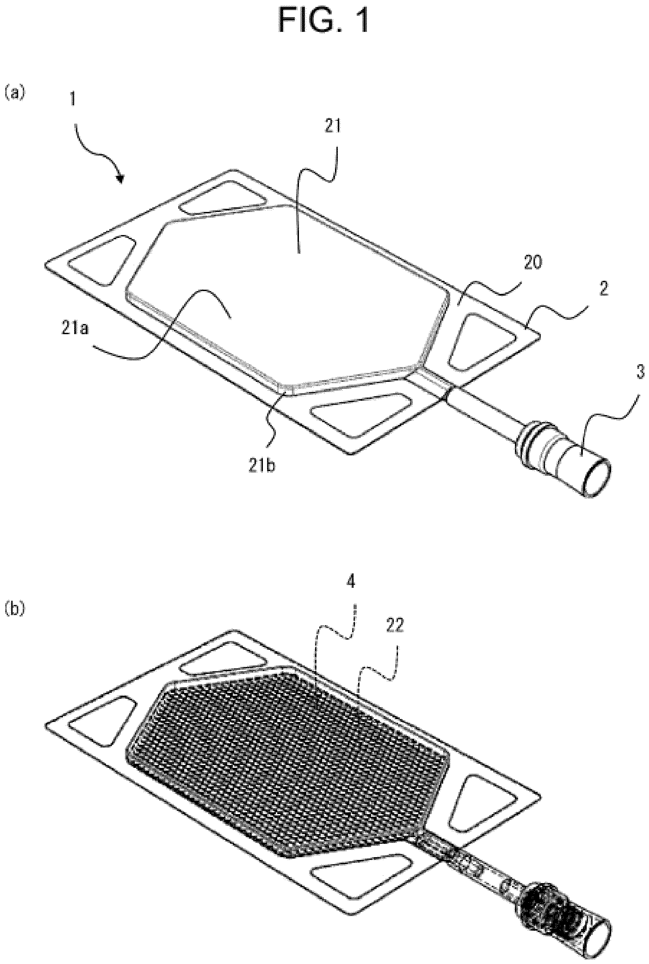

Figure 1 shows one example of the cell culture bag according to the present invention. A cell culture bag 1 has a sac-like configuration that comprises: a bag body 2 formed by stacking an upper face film 21 constituting the upper face and a lower face film 22 constituting the lower face, and sealing a peripheral part 20; and a port 3. A plurality of recesses 4 arranged in a staggered pattern are formed and retained by the lower face film 22 by a molding technique such as heat pressure molding. The upper face film 21 constituting the upper face has a bulging shape having a substantially flat top face portion 21a that covers over the entire plurality of recesses 4, and a sloping portion 21b around the top face portion 21a, and can thereby be sealed with the lower face film 22 to form the cell culture bag having a thickness. When the bag is filled with a medium or when a pressure is applied to the bag, deformation that lifts the periphery of the lower face film 22 can be suppressed.

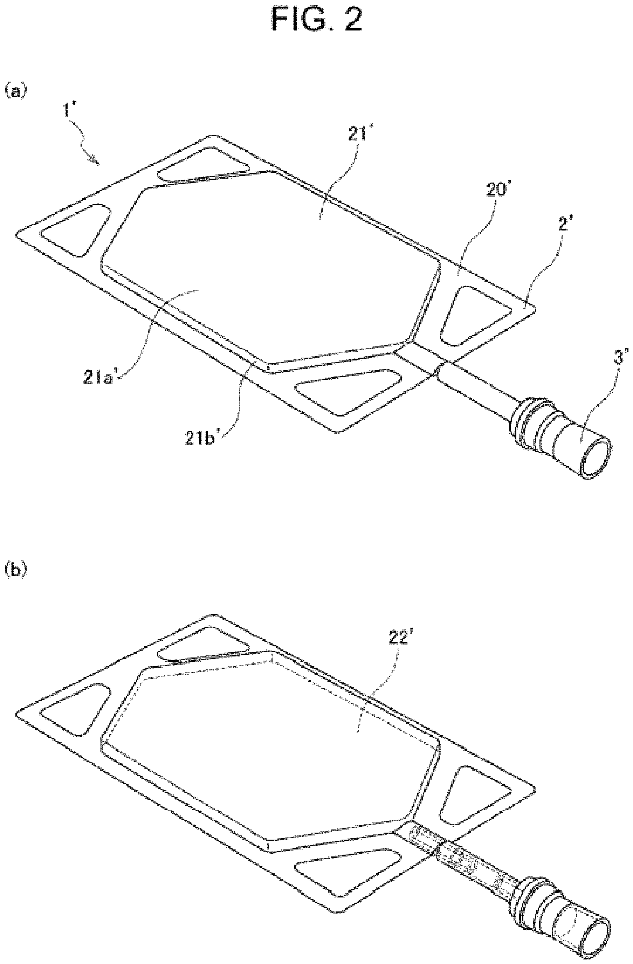

Figure 2 shows another example of the cell culture bag according to the present invention. In a cell culture bag 1', recesses are not directly formed in a lower face film 22' and are patterned after the shape of a placement face of a placement table contacted with the lower face film 22' following placement of the cell culture bag 1' on the placement table.

The placement table for the cell culture bag 1' is a plate-like member having a flat placement face for placing the cell culture bag, and depressions for forming recesses on the lower face of the cell culture bag. The placement table can be formed from a metal, a hard resin, or the like. The depressions of the placement face can have a shape capable of forming the recesses mentioned above on the lower face of the cell culture bag pressed thereagainst. The depressions may be formed by establishing corresponding recesses and/or projections on the placement face, or through-holes appropriately arranged in the placement face may be used as the depressions.

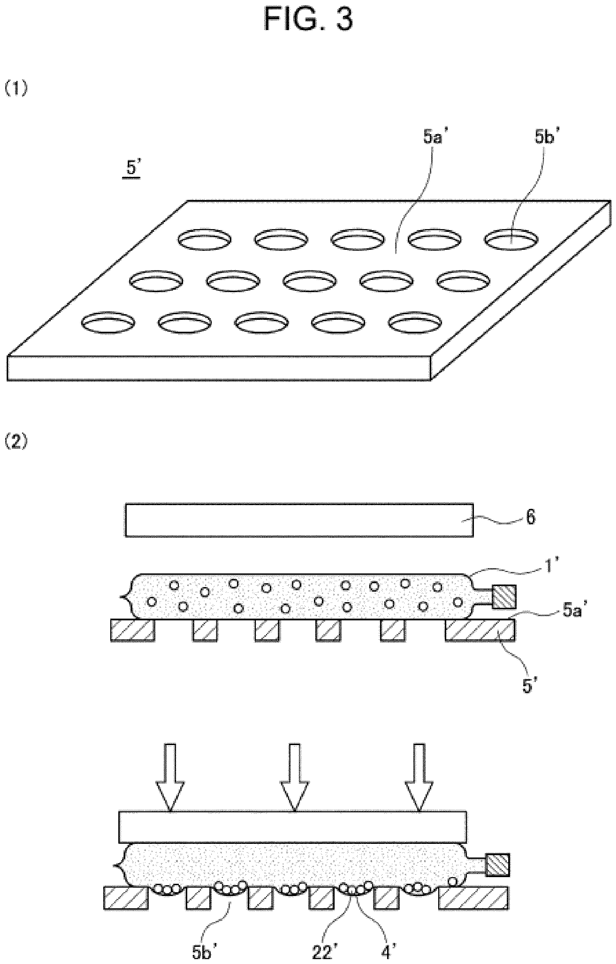

Figure 3(1) shows one example of a placement table 5' which forms recesses in the cell culture bag 1'. The placement table 5' has a flat placement face 5a' for placing the cell culture bag 1', and depressions 5b' in the form of through-holes for forming recesses on the lower face of the cell culture bag 1'. As shown in Figure 3(2), the cell culture bag 1' packed with cells and a predetermined culture solution is placed on the placement table 5'. Then, the cell culture bag 1' is sandwiched between a pressing member 6 mentioned later and the placement table 5', and a pressure is applied thereto so that the lower face film 22' is pressed against the placement face 5a and partially protruded from the portions of the depressions 5b' to form recesses 4'.

### 2. Culture apparatus

In the present invention, a culture apparatus having a configuration capable of applying a pressure to the cell culture bag packed with cells and a predetermined culture medium can be used. The culture apparatus has at least: a placement table on which the cell culture bag is to be placed; a pressing member which holds the upper face of the cell culture bag; a support mechanism which supports the up-and-down movement of one or both of the placement table and the pressing member; a pressure application unit which presses one or both of the placement table and the pressing member against the cell culture bag; and a liquid sending unit which injects or discharges cells or a medium via the port of the cell culture bag. In the case of applying a pressure to the cell culture bag by the self-weight of the pressing member, the pressure application unit may be omitted. Examples of the liquid sending unit include, but are not limited to, pumps such as peristaltic pumps, and syringes. The culture apparatus may be portable so as to be transferred to an incubator for use, or may be installed in an incubator.

As used herein, the phrase "applying a pressure" means that the liquid depth of the cell culture bag packed with cells and a predetermined culture medium is equalized. The cell culture bag packed with cells and a predetermined culture medium does not have a uniform liquid depth without applying a pressure, because the liquid depth has a relatively high portion and a relatively low portion in a mixed manner due to "kink", "wrinkle", "bending", "warpage", etc. formed in the flexible upper face film and/or lower face film. Such "kink", "wrinkle", "bending", "warpage", etc. formed in the upper face film and/or the lower face film can be smoothed out by "applying a pressure". Thus, the liquid depth of the cell culture bag can be equalized. The step of "applying a pressure" can be performed by sandwiching the cell culture bag packed with cells and a predetermined culture medium between the upper face (placement face) of the placement table and the lower face of the pressing member. Specifically, the pressing member can be pushed down toward the cell culture bag through the use of the support mechanism and the pressure application unit to apply a pressure to the cell culture bag from above, and/or the placement table can be pushed up toward the cell culture bag through the use of the support mechanism and the pressure application unit to apply a pressure to the cell culture bag from below. Alternatively, such application of a pressure may be omitted, and instead, a pressure can applied to the cell culture bag by the self-weight of the pressing member. By such an approach, "kink", "wrinkle", "bending", "warpage", etc. formed in the upper face film and/or the lower face film can be smoothed out at least in a region contacted with the lower face of the pressing member, and the liquid depth of the cell culture bag can be equalized.

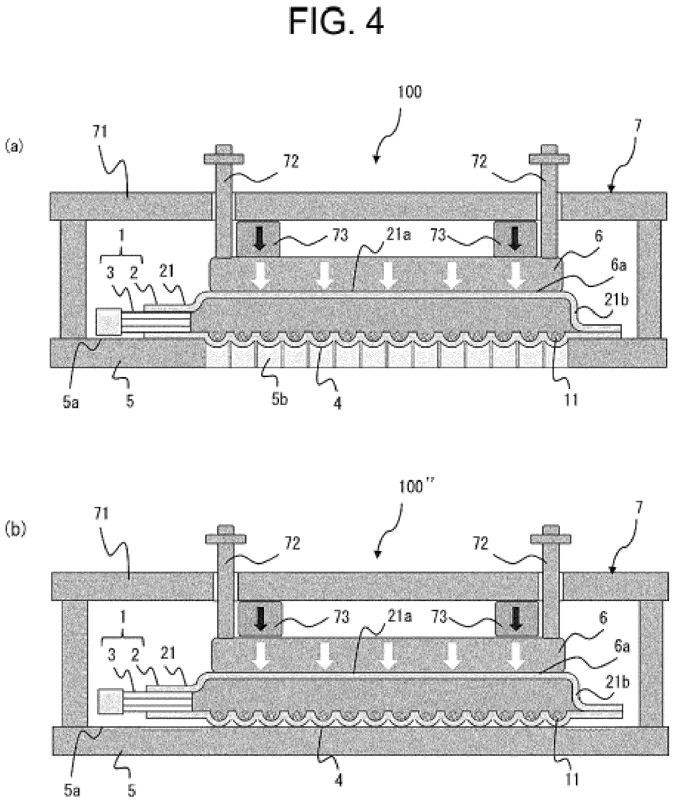

Figure 4 shows a cross-sectional schematic view of one example of a culture apparatus that can be used in the present invention. A culture apparatus 100 or 100" of Figure 4 has a configuration that applies a pressure by pushing down a pressing member 6 from above toward a cell culture bag 1 placed on a placement table 5.

The culture apparatus 100 or 100'' has: a placement table 5 on which the cell culture bag 1 is to be placed; a pressing member 6 having a bottom face 6a which presses a top face portion 21a of an upper face film 21; and a support mechanism 7 which supports the pressing member 6.

In Figure 4, the liquid sending unit which injects or discharges cells or a medium from a port 3 of the cell culture bag 1 is not shown.

The placement table 5 has a placement face 5a on which the cell culture bag 1 is horizontally placed. This placement face 5a can be a flat face (Figure 4(b)), or openings 5b which accept a plurality of recesses 4 formed in a lower face film 22 of the cell culture bag 1 may be formed (Figure 4(a)). In the presence of the openings 5b, the placement face 5a supports the lower face film 22 without coming into contact with the recesses 4. Therefore, the collapse or deformation of the recesses 4 is circumvented, and cells can be prevented from leaking out of the recesses 4. Furthermore, gas permeability through the lower face film 22 is enhanced. The shape of the openings 5b is not limited to such openings and may be, for example, recesses or may be a wire-mesh shape. Each opening 5b may have not only a shape that accepts each individual recess 4 but a shape capable of accepting a plurality of recesses 4 as long as the collapse or deformation of the recesses 4 can be circumvented.

The pressing member 6 is a plate-like member having a planar shape tailored to the top face portion 21a of the cell culture bag 1, and has a flat bottom face 6a.

The support mechanism 7 is constituted by: a frame 71 disposed on the placement face 5; guide pins 72 which extend upward from four corners of the upper face of the pressing member 6 and penetrate the frame 71 so as to be movable up and down; and pressure application units 73 which push down the pressing member 6. The pressure application units 73 apply a pressure to the cell culture bag 1 by pushing down the pressing member 6 toward the cell culture bag 1. The pressure to be applied can be adjusted by adjusting the force of the pressure application units 73 to push down the pressing member 6, and the distance travelled by the pressing member pushed down. The pressure application units 73 can have a configuration capable of pushing down the pressing member 6 by a predetermined force or at a predetermined distance, and can have a configuration made of screws, springs, magnets, or a combination thereof.

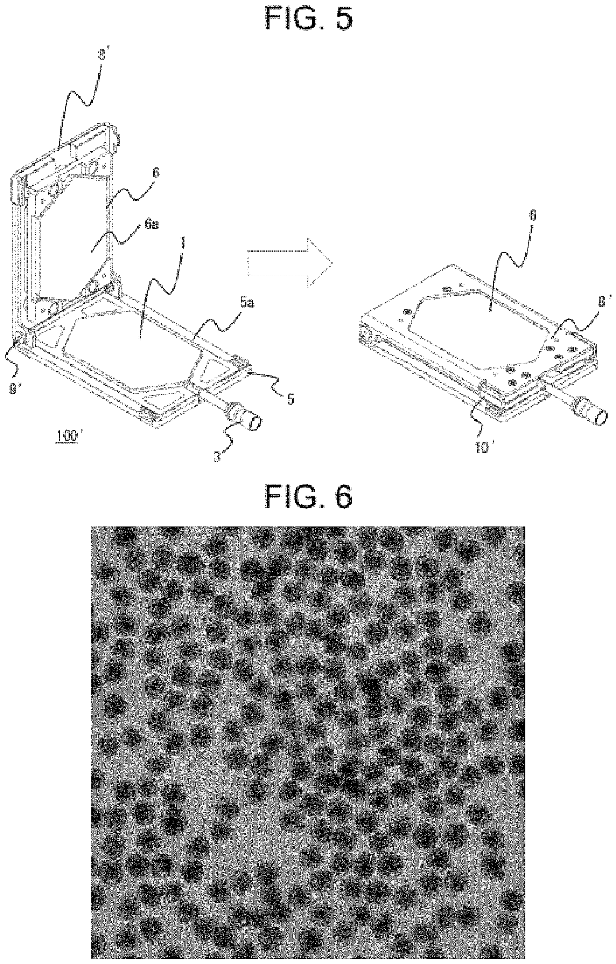

Figure 5 is a perspective view showing another example of a culture apparatus that can be used in the present invention. A culture apparatus 100' of Figure 5 has a placement table 5 having a placement face 5a, and a top cover 8' connected to this placement table 5 via a hinge 9'. The top cover 8' is configured so as to be openable and closable with the hinge 9' as an axis. The top cover 8' is constituted by: a pressing member 6 having a flat bottom face 6a tailored to a top face portion 21a of a cell culture bag 1; guide pins (not shown) which extend upward from four corners of the upper face of the pressing member 6 and penetrate the top cover 8' so as to be movable up and down; and pressure application units (not shown) which push down the pressing member 6 toward the cell culture bag 1.

When the cell culture bag 1 is housed in the culture apparatus 100', the top cover 8' is first opened via the hinge 9' (Figure 5(a)). In this state, the cell culture bag 1 is then placed on the placement face 5a having openings (not shown) that accept a plurality of recesses. The top cover 8' is then closed as shown in Figure 5(b) and maintained by lock mechanism 10'. A pressure can be applied to the cell culture bag 1 by the pressure application units which push down the pressing member 6 until a predetermined pressure is applied.

Both Figures 4 and 5 described above show examples using the cell culture bag 1. In the case of using a cell culture bag 1', a culture apparatus having the same configuration as that of the culture apparatus mentioned above can be used in each case except that a placement table having a placement face 5a' capable of forming recesses in the cell culture bag 1', such as a placement table 5', can be used as the placement table.

### 3. Cell

In the present invention, examples of the cells for use in the production of cell aggregates include, but are not particularly limited to, pluripotent stem cells and differentiation-induced cells thereof, neural stem cells, hepatocytes, corneal stem cells, pancreatic islet cells, mesenchymal stem cells, adipose stem cells, fibroblasts, myoblasts, chondrocytes, and vascular endothelial cells. A plurality of cells may be used as a mixture to form cell aggregates as organoids.

In the present invention, the "pluripotent stem cells" refer to embryonic stem cells (ES cells) and cells potentially having pluripotent differentiation similar thereto, i.e., the ability to differentiate into various tissues (all the endoderm, the mesoderm, and the ectoderm) of a living body. Examples of the cells having pluripotent differentiation similar to that of the ES cells include "induced pluripotent stem cells" (in the present specification, also referred to as "iPS cells"). In the present invention, the pluripotent stem cells are preferably human pluripotent stem cells. The "induced pluripotent stem cells" refer to cells that are obtained by introducing mammalian somatic cells or undifferentiated stem cells with particular factors (nuclear reprogramming factors) for reprogramming. Various "induced pluripotent stem cells" are currently available, and iPS cells established by Yamanaka et al. by introducing mouse fibroblasts with four factors, Oct3/4, Sox2, Klf4 and c-Myc (Takahashi K and Yamanaka S., Cell, (2006) 126: 663-676) as well as human cell-derived iPS cells established by introducing human fibroblasts with these four factors (Takahashi K, Yamanaka S., et al., Cell, (2007) 131: 861-872.), Nanog-iPS cells established by introduction with these four factors followed by screening with the expression of Nanog as an index (Okita, K., Ichisaka, T., and Yamanaka, S. (2007). Nature 448, 313-317.), iPS cells prepared by a method free of c-Myc (Nakagawa M, Yamanaka S., et al., Nature Biotechnology, (2008) 26, 101-106)), or iPS cells established by introduction with 6 factors in a virus-free manner (Okita K et al., Nat. Methods 2011 May; 8 (5): 409-12; and Okita K et al., Stem Cells. 31 (3): 458-66) may be used. Alternatively, induced pluripotent stem cells prepared by Thomson et al. and established by introduction with four factors, OCT3/4, SOX2, NANOG, and LIN28 (Yu J., Thomson JA. et al., Science (2007) 318: 1917-1920), induced pluripotent stem cells prepared by Daley et al. (Park IH, Daley GQ. et al., Nature (2007) 451: 141-146), induced pluripotent stem cells prepared by Sakurada et al. (Japanese Patent Laid-Open No. 2008-307007), or the like may be used. In addition, any of induced pluripotent stem cells known in the art, described in all published articles (e.g., Shi Y., Ding S., et al., Cell Stem Cell, (2008) Vol 3, Issue 5, 568-574; Kim JB., Scholer HR., et al., Nature, (2008) 454, 646-650; and Huangfu D., Melton, DA., et al., Nature Biotechnology, (2008) 26, No 7, 795-797) or patents (e.g., Japanese Patent Laid-Open No. 2008-307007, Japanese Patent Laid-Open No. 2008-283972, US2008/2336610, US2009/047263, WO2007/069666, WO2008/118220, WO2008/124133, WO2008/151058, WO2009/006930, WO2009/006997, and WO2009/007852) can be used.

The "differentiation-induced cells" of the pluripotent stem cells mentioned above mean cells that are obtained by inducing the differentiation of the pluripotent stem cells and characterized by a predetermined phenotype or the expression of a marker. The "marker" means a cell antigen specifically expressed from a predetermined cell type, or a gene thereof, such as "marker protein" or "marker gene". Preferably, the marker is a cell surface marker. In this case, the condensation, isolation, and/or detection of live cells can be carried out. The marker may be a positive selection marker or a negative selection marker.

The marker protein can be detected by use of immunological assay (ELISA, immunostaining, flow cytometry, etc.) using an antibody specific for the marker protein. The marker gene can be detected by use of a nucleic acid amplification method and/or a nucleic acid detection method (RT-PCR, microarray, biochip, etc.) known in the art. For example, the term "positive" to the marker protein means that the marker protein is detected as being positive by flow cytometry. The term "negative" to the marker protein means a level equal to or lower than a detection limit in flow cytometry. The term "positive" to the marker gene means that the marker gene is detected by RT-PCR. The term "negative" to the marker gene means a level equal to or lower than a detection limit in RT-PCR.

Examples of the differentiation-induced cells that can be used in the present invention include insulin-secreting cells obtained by inducing the differentiation of pluripotent stem cells. The insulin-secreting cells include insulin-producing cells and/or pancreatic β cells.

The "insulin-producing cells" or the "pancreatic β cells" are cells differentiation-induced from pluripotent stem cells in accordance with an approach known in the art (WO2009/012428; WO2016/021734; and Stem Cell Research (2015) 14, 185-197). The insulin-producing cells are cells characterized by the expression of at least one marker selected from insulin and NK6 homeobox 1 (NKX6.1). The pancreatic β cells are cells matured from the insulin-producing cells and are characterized by the expression of at least one marker selected from MAFA, UCN3, and IAPP.

The cells that can be used in the present invention may be cells collected from a living body, may be cultured cells, or may be frozen-thawed cells of these cells. The cells are used in a dissociated or dispersed state.

### 4. Culture of cell and recovery of cell aggregate

The cells are added, together with a culture medium, to the cell culture bag, stirred, and cultured in the bag in a state where a pressure is applied to the bag.

The culture medium can be used by appropriately selecting a suitable one for the cells used. The cells and the culture medium are added from the port of the cell culture bag using a liquid sending unit such as a pump (e.g., a peristaltic pump) or a syringe, and stirred. By such stirring, the numbers of cells that enter the individual recesses on the lower face of the cell culture bag can be substantially equalized in a subsequent step. The stirring can be performed one or more times at any timing before application of a pressure to the cell culture bag. The stirring can be performed, for example, before or after placement of the cell culture bag on the placement table of the culture apparatus, or immediately before application of a pressure to the cell culture bag by the culture apparatus. The stirring may be performed manually or by a robot arm and/or can be performed using a vibrator.

The number of cells added to the cell culture bag is appropriately adjustable depending on a factor such as the size of the cell culture bag, the size or number of the recesses on the lower face of the cell culture bag, the type of the cells used, or the desired size of cell aggregates, and is not particularly limited. The number is in the range of preferably 1 × 10⁵ to 1 × 10⁷ cells/mL, more preferably 5 × 10⁵ to 5 × 10⁶ cells/mL, and/or in the range of 1 × 10⁴ to 5 × 10⁶ cells/cm², more preferably 1 × 10⁵ to 2 × 10⁶ cells/cm². If the number of cells added is too smaller or too larger than this range, a large quantity of cell aggregates having a uniform size may not be able to be produced.

The amount of the culture medium added to the cell culture bag is appropriately adjustable depending on a factor such as the size of the cell culture bag, the number of cells added to the cell culture bag, a culture period, or the desired size of cell aggregates, and is not particularly limited. The amount can be an amount that allows the liquid depth (distance from the inner surface of a flat face (site except for the recesses) of the lower face film that comes in contact with a culture solution to the inner surface of the upper face film) of the culture medium added to the cell culture bag to be on the order of preferably 1 mm to 20 mm, more preferably 2 mm to 8 mm, in applying a pressure to the cell culture bag. If the amount of the culture medium added to the cell culture bag is large at the time of cell inoculation, the liquid depth is elevated. Such elevated liquid depth requires more time for the precipitation of the cells, during which the cells may move in the horizontal direction due to thermal convection or the like and be not precipitated with a uniform concentration. In such a case, the numbers of cells that enter the individual recesses may not be able to be equalized. In this case, the amount of the culture medium added at the time of cell inoculation may be decreased, and a fresh culture medium can be further added after formation of the cell aggregates.

The application of a pressure to the cell culture bag can be performed using the culture apparatus mentioned above, and can be performed by sandwiching the cell culture bag between the placement table with the cell culture bag placed thereon and the pressing member that faces the top face portion of the cell culture bag.

The amplitude of the pressure to be applied to the cell culture bag by the culture apparatus can be an amplitude that can smooth out "kink", "wrinkle", "bending", "warpage", etc. formed in the flexible upper face film that faces the pressing member, and/or the lower face film, and is capable of equalizing the liquid depth of the cell culture bag. The amplitude is appropriately adjustable depending on a factor such as the size or depth of the cell culture bag, the material of the cell culture bag, or the amount of the culture medium added to the cell culture bag, and is not particularly limited. The value can be selected from the range of preferably 0.001 to 0.1 kgf/cm², more preferably 0.002 to 0.05 kgf/cm². If the amplitude of the pressure to be applied is too small, "kink", "wrinkle", "bending", "warpage", etc. formed in the upper face film and/or the lower face film cannot be smoothed out and the liquid depth of the cell culture bag may not be able to be equalized. Thus, the particle size of the finally obtained cell aggregates may not be able to be substantially equalized. On the other hand, if the amplitude of the pressure to be applied is too large, the cell culture bag may be broken or the survival or proliferation of the cells may be influenced.

The cells are cultured while the application of a pressure to the cell culture bag is maintained by the culture apparatus. This keeps the liquid depth uniform, and such a uniform liquid depth can substantially equalize the numbers of cells that enter the recesses and/or can substantially equalize medium components. The cells enter the recesses where the aggregation of the cells in turn progresses to form cell aggregates. The culture conditions depend on the cells used, and the culture can be performed in an incubator adjusted to an appropriate temperature (30 to 40°C, for example, 37°C), CO₂ concentration (5 to 10%, for example, 5%), and humidity (90 to 95%, for example, 95%). In the case of transferring the culture apparatus having the cell culture bag into an incubator, this may be performed manually or by a robot arm. The culture period can be appropriately determined depending on a factor such as the desired size of cell aggregates or the type of the cells, and is not particularly limited. The culture period is, for example, 1 to 10 days, preferably 2 to 7 days. The culture medium may be replaced, if necessary, with a fresh one.

Subsequently, the applied pressure is released after the completion of culture. Then, the formed cell aggregates are recovered. The formed cell aggregates can be recovered by use of an arbitrary approach capable of floating the cell aggregates in the culture medium and taking the cell aggregates out of the recesses. A physical approach can be used as such an approach. For example, an approach of flipping the cell culture bag vertically, applying vibration to the cell culture bag, thrusting up the recesses from the outer side of the cell culture bag using, for example, a protruding member (Japanese Patent Laid-Open No. 2019-118319), or removing the cell culture bag from the placement face and flattening the recesses (when the recesses are formed from the depressions of the placement face) can be used. Such an approach may be performed manually or by a robot arm and/or can be performed using a vibrator. Alternatively, air, a culture medium, or an appropriate buffer solution such as saline may be injected to the cell culture bag via the port of the cell culture bag, and the cell aggregates can be floated by the resulting jet flow and taken out of the recesses. The cell aggregates floated in the culture solution can be recovered, together with the culture medium, etc., from the port of the cell culture bag.

### 5. Cell aggregate

The method of the present invention can conveniently produce a large quantity of cell aggregates having a substantially uniform particle size.

The number of cell aggregates produced can be adjusted on the basis of the number of recesses on the lower face of the cell culture bag, and is not particularly limited. The number can be preferably 100000 to 1000000 or 300000 to 1000000.

The phrase "having a substantially uniform particle size" means that 70% or more, preferably 80% or more, more preferably 90% or more, further preferably 95% or more, of the produced cell aggregates have a particle size within ±10%, preferably within ±5%, of the median particle size of the cell aggregates. The particle size and particle size distribution of the cell aggregates can be measured using one or two or more in combination of conventional approaches known in the art, for example, measurement using microscopic observation, a dynamic light scattering method, laser diffractometry, a centrifugal precipitation method, FFF, and an electric sensing zone method.

The size of the cell aggregates to be produced may vary depending on a factor such as the type or number of the cells used, the size of the cell culture bag, the size or number of the recesses on the lower face of the cell culture bag, or the culture period. For example, in one embodiment, 700 or more, preferably 80% or more, more preferably 90% or more, further preferably 95% or more, of the produced cell aggregates can have a particle size of 100 µm to 200 µm, preferably 120 µm to 180 µm, more preferably 140 µm to 160 µm. If the size of the cell aggregates is larger than this range, large stress may be placed on the cells or the number of apoptotic cells may be increased. Thus, the formation of the cell aggregates might be hindered.

The number of cells included in each cell aggregate may vary depending on a factor such as the type or number of the cells used, the size of the cell culture bag, the size or number of the recesses on the lower face of the cell culture bag, or the culture period, and is on the order of 200 to 2000 per cell aggregate.

The produced cell aggregates can be transplanted into a living body of an animal and used in cell transplantation therapy. The produced cell aggregates are transplanted, either directly or after being encapsulated or gelled together with a biodegradable hydrogel, to an affected part and is thereby useful as a cell medicine for treating a disease.

The produced cell aggregates may be used as a prodrug. The "prodrug" refers to a cell population that is converted to cells having a function of treating a disease by differentiation after transplantation into a living body.

The produced cell aggregates may be administered, either directly or as a pharmaceutical composition after being mixed with a pharmacologically acceptable carrier, etc., to a subject to be treated.

Hereinafter, the present invention will be described with reference to Examples. However, the present invention is not limited by these Examples.

### Examples

### Example 1: Production of cell aggregate using insulin-producing cell

### 1. Preparation of insulin-producing cell

The insulin-producing cells were prepared by inducing the differentiation of human iPS cells in accordance with a conventional approach known in the art (WO2009/012428; WO2016/021734; and Stem Cell Research (2015) 14, 185-197)).

The human iPS cells used were the Ff-I14s04 line established by Kyoto University.

In inducing the differentiation, first, undifferentiated iPS cells were inoculated to a bioreactor. The iPS cells maintained on a culture dish were treated with an EDTA solution and dissociated into single cells. Subsequently, the iPS cells dispersed in a medium were inoculated at a density of 6 × 10⁶ cells per bioreactor and suspension-cultured by stirring at 37°C. The culture solution used for inoculation was StemFit AK03N medium supplemented with 10 µM Y-27632. The cells were cultured for 1 day to form cell spheroids.

Next, the iPS cells were induced to differentiate into endodermal cells. The medium was replaced with Dulbecco's modified Eagle medium (Thermo Fisher Scientific, Inc.) containing activin A (10 ng/mL) (PeproTech, Inc.), a GSK3β inhibitor CHIR99021 (3 µM) (Axon Medchem), 1% dimethyl sulfoxide (FUJIFILM Wako Pure Chemical Corp.), 1% B-27(R) (Thermo Fisher Scientific, Inc.), and 0.1% Pluronic(R) F68 (Sigma-Aldrich Co., LLC), and the cells were cultured for 1 day and subsequently cultured for 2 days using Dulbecco's modified Eagle medium containing activin A (10 ng/mL) (PeproTech, Inc.), 1% dimethyl sulfoxide (FUJIFILM Wako Pure Chemical Corp.), insulin-free B-27(R) (Thermo Fisher Scientific, Inc.), and 0.1% Pluronic F68 (Sigma-Aldrich Co., LLC).

Next, the endodermal cells were induced to differentiate into endocrine progenitor cells in accordance with a conventional approach known in the art (WO2009/012428; WO2016/021734; and Stem Cell Research (2015) 14, 185-197) and (Nature Biotechnology 2014; 32: 1121-1133)).

Next, the endocrine progenitor cells were induced to differentiate into insulin-producing cells. The endocrine progenitor cells were cultured for 4 days in Iscove's modified Eagle medium-option Zn⁺⁺ containing 1% B-27(R), L-ascorbic acid phosphate magnesium salt (58 mg/L) (FUJIFILM Wako Pure Chemical Corp.), activin receptor-like kinase 5 inhibitor II (10 µM) (Santa Cruz Biotechnology, Inc.), LDN-193189 (100 nM) (MedChemExpress), triiodothyronine (1 µM) (Sigma-Aldrich Co., LLC), RO4929097 (1 µM) (Selleck Chemicals LLC) and 0.1% Pluronic F68 (Sigma-Aldrich Co., LLC), and for 3 days in a medium further supplemented with PD-166866 (1 µM) (Sigma-Aldrich Co., LLC) to obtain insulin-producing cells. The obtained insulin-producing cells were dispersed into single cells using TrypLE(R) Select (Thermo Fisher Scientific, Inc.).

### 2. Production of cell aggregate

The cell culture bag used was a prepared cell culture bag having the same configuration as that of the cell culture bag shown in Figure 1. Specifically, the cell culture bag has a configuration that was prepared by stacking two gas permeable flexible polyethylene films each having a thickness of 100 µm, and sealing their peripheries, and had a port. The lower face film of the cell culture bag had 4.5 × 10⁵ recesses each having a depth of 150 µm and a diameter of 350 µm on a 700 cm² lower face (642 recesses/cm² per lower face unit area), and the upper face film thereof had a bulging shape having a 4 mm high bulge. The inner faces of the films were subjected to low cell adhesion coating with a phospholipid polymer.

The culture apparatus used was the culture apparatus shown in Figure 5.

The prepared insulin-producing cells (1.25 × 10⁶ cells/mL) mentioned above were enclosed, together with a medium (MCDB131/20 mM glucose/sodium bicarbonate/fatty acid-free bovine serum-derived albumin/ITS-X/Glutamax/ascorbic acid/penicillin-streptomycin)) containing differentiation factors (triiodothyronine, activin receptor-like kinase 5 inhibitor II, zinc sulfate, heparin, N-acetylcysteine, Trolox, and R428) of a conventional approach known in the art (Nature Biotechnology 2014; 32: 1121-1133)) as well as a ROCK inhibitor (Y27632, etc.), a fibroblast growth factor receptor inhibitor (PD-166866, etc.), and the like, in the cell culture bag. The port was closed, and the cell culture bag was inverted and placed flat such that the recesses were turned up. The contents in the bag were stirred by holding both hands against the bag from above and alternately pressing the bag by the hands. Subsequently, the cell culture bag was flipped immediately after stirring such that the recesses were turned down. The bag was placed on the placement table of the culture apparatus, and a pressure of 2 kgf was applied from the upper face of the bag by the pressing member. In this form, the culture apparatus was transferred into an incubator, followed by culture at 37°C for 4 days under 5% CO₂ conditions.

After the completion of culture, the cell culture bag was taken out of the culture apparatus and flipped over so that each cell aggregate formed in each recess was floated into the culture medium from the recess. A syringe was connected to the port, and the cell aggregates were recovered, together with the culture medium, from the port. Only slight cell aggregates remained in the cell culture bag, and most cell aggregates were able to be recovered.

Figure 6 shows a photographic view of the recovered cell aggregates. The cells in the recovered cell aggregates were analyzed for the expression of a marker protein by flow cytometry and consequently found to include 41.6% cells co-expressing insulin and NKX6.1, confirming the production of insulin-producing cells.

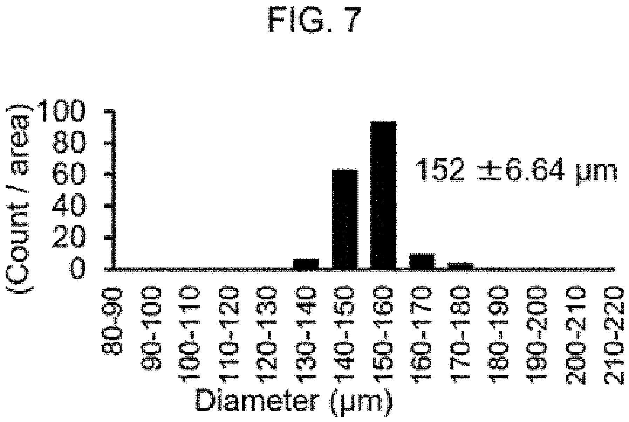

The whole amount of the recovered culture medium containing the cell aggregates was transferred to a 175 cm² flask, and the cell aggregates were photographed with a camera attached to a phase contrast microscope. The particle size of each cell aggregate was measured from the image. The results are shown in Figure 7.

70% or more of the recovered cell aggregates had a particle size within ±10% of the median particle size of the cell aggregates and had a particle size of 140 µm to 160 µm.

From these results, the method of the present invention was confirmed to be able to simply produce a large quantity of cell aggregates having a substantially uniform particle size.

### Example 2: Production of cell aggregate using iPS cell

### 1. Preparation of iPS cell

Human iPS cells (1231A3 line) were subcultured in accordance with the protocol published from the Center for iPS Cell Research and Application (CiRA), Kyoto University, detached with TrypLE + EDTA, and suspended in StemFit medium (Ajinomoto Co., Inc.) supplemented with 1% ROCK inhibitor (Y27632).

### 2. Production of cell aggregate

The cell culture bag used was a prepared cell culture bag having the same configuration as that of the cell culture bag shown in Figure 1. Specifically, the cell culture bag has a configuration that was prepared by stacking two gas permeable flexible polyethylene films each having a thickness of 100 µm, and sealing their peripheries, and had a port. The lower face film of the cell culture bag had 1.8 × 10⁴ recesses each having a depth of 200 µm and a diameter of 500 µm on a 50 cm² lower face (360 recesses/cm² per lower face unit area), and the upper face film thereof had a bulging shape having a 4 mm high bulge. The inner faces of the films were subjected to low cell adhesion coating with a phospholipid polymer.

20 mL (2.25 × 10⁵ cells/mL) of the cell suspension mentioned above was enclosed in the cell culture bag. The port was closed, and the cell culture bag was inverted and placed flat such that the recesses were turned up. The suspension in the bag was stirred by holding both hands against the bag from above and alternately pressing the bag by the hands. Subsequently, the cell culture bag was flipped immediately after stirring such that the recesses were turned down. The bag was placed on the placement table of the culture apparatus, and a pressure of 2 kgf was applied from the upper face of the bag by the pressing member. On the other hand, the same operation was performed as to a control without applying a pressure except that this pressure of 2 kgf was not applied. In this form, the culture apparatus was transferred into an incubator, followed by culture at 37°C for 2 days under 5% CO₂ conditions.

After the completion of culture, the cell culture bag was taken out of the culture apparatus and flipped over so that each cell aggregate formed in each recess was floated into the culture medium from the recess. A syringe was connected to the port, and the culture medium containing the cell aggregates was recovered from the port.

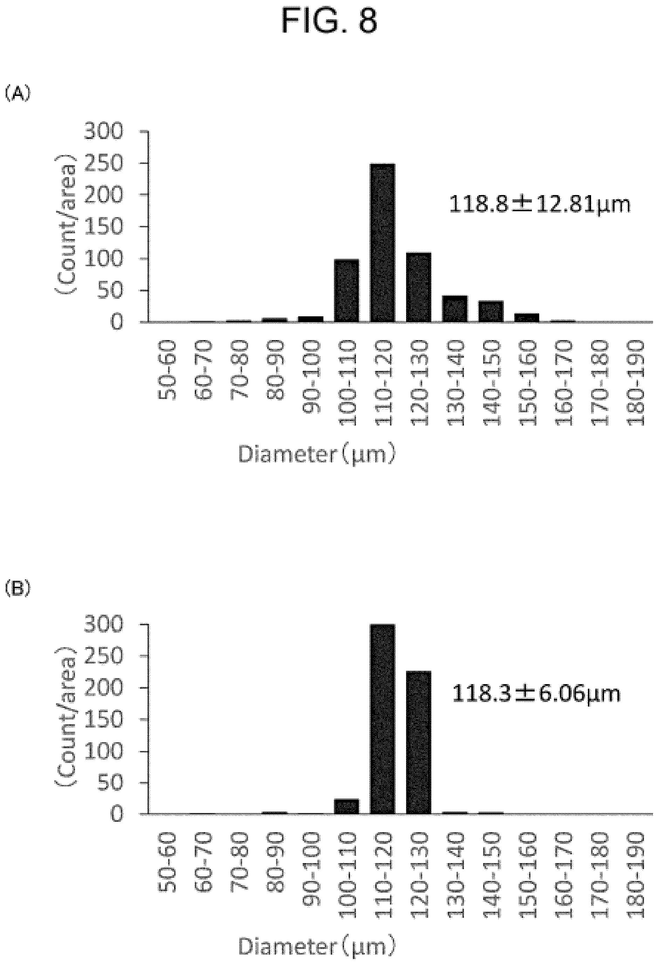

The whole amount of the recovered culture medium containing the cell aggregates was transferred to a 10 cm Petri dish, and the cell aggregates were photographed with a camera attached to a phase contrast microscope. The particle size of each cell aggregate was measured from the image. The results are shown in Figure 8.

The deviation of the particle size was compared between the recovered cell aggregates. As a result, the cell aggregates produced by culture without applying a pressure had a particle size on the order of ±10% of the center value, whereas the cell aggregates obtained by culture while a pressure was applied had a particle size on the order of ±5% of the center value.

From these results, the method of the present invention was confirmed to be able to simply produce a large quantity of cell aggregates having a substantially uniform particle size.

### Reference Signs List

1 and 1': Cell culture bag
2 and 2': Bag body
20 and 20': Peripheral part
21 and 21': Upper face film
21a and 21a': Top face portion
21b and 21b': Sloping portion
22 and 22': Lower face film
3 and 3': Port
4 and 4' Recess
5 and 5': Placement table
5a and 5a': Placement face
5b: Opening
5b': Depression
6: Pressing member
6a: Bottom face
7: Support mechanism
71: Frame
72: Guide pin
73: Pressure application unit
8': Top cover
9': Hinge
10': Lock mechanism
100, 100', and 100'': Culture apparatus
11: Cell aggregate

## Claims

1. A method for producing cell aggregates using a cell culture bag,
the cell culture bag having an upper face and a lower face and comprising a plurality of recesses on the lower face,
the method comprising the steps of:
(1) adding cells and a medium to the cell culture bag, stirring the contents, and culturing the cells while applying a pressure in at least one direction to the cell culture bag; and
(2) recovering the formed cell aggregates after the completion of culture.

2. The method according to claim 1, wherein 100000 to 1000000 cell aggregates are recovered.

3. The method according to claim 1 or 2, wherein the recovered cell aggregates have a substantially uniform particle size.

4. The method according to any one of claims 1 to 3, wherein 70% or more of the recovered cell aggregates have a particle size within ±10% of the median particle size of the cell aggregates.

5. The method according to any one of claims 1 to 4, wherein 70% or more of the recovered cell aggregates have a particle size of 140 µm to 160 µm.

6. The method according to any one of claims 1 to 5, wherein 200 to 2000 cells are included per recovered cell aggregate.

7. The method according to any one of claims 1 to 6, wherein the cells are frozen-thawed cells.

8. The method according to any one of claims 1 to 7, wherein the cells are insulin-secreting cells.

9. The method according to any one of claims 1 to 8, wherein the cell aggregates are recovered by flipping the cell culture bag vertically.

10. The method according to any one of claims 1 to 9, wherein the cell aggregates are recovered by injecting air to the cell culture bag.

11. The method according to any one of claims 1 to 10, wherein the step of culturing the cells while applying a pressure in at least one direction to the cell culture bag is the step of culturing the cells while applying a pressure to the cell culture bag from above, from below or from both above and below.

12. The method according to claim 11, wherein the step of culturing the cells while applying a pressure in at least one direction to the cell culture bag is the step of culturing the cells while applying a pressure to the cell culture bag from above.

13. The method according to any one of claims 1 to 12, wherein a depth:diameter ratio of the recesses is 1:1.5 to 2.5.

14. The method according to any one of claims 1 to 13, wherein the recesses have a spherical cap shape.

15. The method according to any one of claims 1 to 14, wherein a pitch of the recesses is 0.35 mm to 0.49 mm.

16. The method according to any one of claims 1 to 15, wherein a depth of the recesses is 150 µm to 220 µm.

17. The method according to any one of claims 1 to 16, wherein a liquid depth of the cell culture bag is 1.5 mm to 6 mm.

18. The method according to any one of claims 1 to 17, wherein the cells are added in an amount of 1 × 10⁵ to 1 × 10⁷ cells/mL to the cell culture bag.

19. The method according to any one of claims 1 to 18, wherein the cells are added in an amount of 1 × 10⁴ to 5 × 10⁶ cells/cm² to the cell culture bag.

20. The method according to any one of claims 1 to 19, wherein a medium is further added after cell aggregate formation.

21. The method according to any one of claims 1 to 20, wherein the lower face comprises 100000 to 1000000 recesses.

22. The method according to any one of claims 1 to 21, wherein the recesses are included at 1 to 1000 recesses/cm² per lower face unit area.

23. A cell aggregate population for transplantation, wherein 70% or more of the cell aggregates have a particle size of 140 µm to 160 µm.

24. The cell aggregate population according to claim 23, comprising 100000 to 1000000 cell aggregates.

25. The cell aggregate population according to claim 23 or 24, comprising insulin-secreting cells.

26. A cell culture bag comprising 100000 to 1000000 cell aggregates having a substantially uniform particle size.

27. The cell culture bag according to claim 26, wherein 70% or more of the cell aggregates have a particle size of 140 µm to 160 µm.

28. The cell culture bag according to claim 26 or 27, wherein the cell aggregates comprise insulin-secreting cells.
